Europäisches Patentamt

(19) European Patent Office    (11) Publication number: **0 117 882**

Office européen des brevets    **B1**

(12) **EUROPEAN PATENT SPECIFICATION**

(45) Date of publication of patent specification: **15.07.87**    (51) Int. Cl.⁴: **C 07 C 103/44,**
    **C 07 D 239/26, C 07 D 239/06**

(21) Application number: **83102156.3**

(22) Date of filing: **04.03.83**

(54) **Novel aminopropylpivalamides and a method of preparation.**

(43) Date of publication of application:
**12.09.84 Bulletin 84/37**

(45) Publication of the grant of the patent:
**15.07.87 Bulletin 87/29**

(84) Designated Contracting States:
**BE CH DE FR GB IT LI NL**

(56) References cited:
**FR-A- 823 138
US-A-3 050 523**

**CHEMICAL ABSTRACTS, vol. 85, (1976) page
462, abstract 142251m Columbus, Ohio, US J.
OKADA et al. "Formation of 2-ethylpyrimidine
from trimethylene-diamine over platinum
group metalaluminum oxide catalysts and its
kinetics study"**

(73) Proprietor: **THE DOW CHEMICAL COMPANY
2030 Dow Center Abbott Road P.O. Box 1967
Midland, MI 48640 (US)**

(72) Inventor: **Pews, Richard Garth
4403 Andre
Midland Michigan 48640 (US)**

(74) Representative: **Weickmann, Heinrich, Dipl.-Ing.
et al
Patentanwälte Dipl.-Ing. H.Weickmann Dipl.-
Phys.Dr. K.Fincke Dipl.-Ing. F.A.Weickmann
Dipl.-Chem. B. Huber Dr.-Ing. H. Liska Dipl.-
Phys.Dr. J. Prechtel Postfach 860820
D-8000 München 86 (DE)**

## Description

The invention relates to novel 3-aminopropylpivalamides and to a method for the preparation thereof.

It has now been found that 3-aminopropylpivalamide can be prepared in high yields by contacting pivalic acid with 1,3-diaminopropane at temperatures above the boiling point of the 1,3-diaminopropane. The unreacted 1,3-diaminopropane is removed by distillation.

The 3-aminopropylpivalamide prepared in this reaction is a novel compound and is advantageously employed to make 2-t-butyltetrahydropyrimidine which is an intermediate in the preparation of compounds having exceptional insecticidal activity.

The 3-aminopropylpivalamide is prepared by reacting pivalic acid with an excess of 1,3-diaminopropane at a temperature of 110° to 240°C, preferably at 200° to 225°C, for 3 to 12 hours. The unreacted 1,3-diaminopropane is removed by distillation.

A 3 to 10 mole excess of 1,3-diaminopropane is usually employed, although larger molar excesses may be used, if desired. If less than a 3 molar excess is utilized, yields tend to be somewhat lower.

The invention is further illustrated by the following examples.

### Example 1

A mixture of pivalic acid (136 g, 1.33 mol) and 1,3-diaminopropane (880 ml, 7.75 mol) was heated overnight at 225°C in a stirred Parr reactor. After cooling and removal of the unreacted 1,3-diaminopropane by distillation, gas chromatographic analysis of the residue, a viscous oily material which was not stable to purification by distillation, showed a 90% yield of 3-aminopropylpivalamide.

### Example 2

A mixture of 25.5 g (0.25 mole) of trimethylacetic acid and 100 ml (112.6 g=1.52 moles) of 1,3-diaminopropane was heated for 16 hours at 200°—210°C.

After cooling and removing the excess 1,3-diaminopropane, the residue, 3-aminopropylpivalamide, a viscous oily material, was analyzed by NMR and gave a spectrum consistent with that expected for 3-aminopropylpivalamide.

## Claims

1. 3-Aminopropylpivalamide.

2. A method for making 3-aminopropylpivalamide which comprises contacting pivalic acid with 1,3-diaminopropane in the absence of a catalyst at tempertures above the boiling point of the 1,3-diaminopropane and removing the excess 1,3-diaminopropane by distillation.

3. Method of Claim 2 wherein the reaction is carried out at 110° to 240°C.

4. Method of Claim 3 wherein the reaction is carried out at 200° to 225°C.

5. Method of Claim 2 wherein the 1,3-diaminopropane is present in from 3 to 10 molar stoichiometric excess.

## Patentansprüche

1. 3-Aminopropylpivalamid.

2. Verfahren zur Herstellung von 3-Aminopropylpivalamid, dadurch gekennzeichnet, daß Pivalinsäure mit 1,3-Diaminopropan in Abwesenheit eines Katalysators bei Temperaturen oberhalb des Siedepunkts des 1,3-Diaminopropans in Kontakt gebracht wird und der Überschuß an 1,3-Diaminopropan durch Destillation entfernt wird.

3. Verfahren nach Anspruch 2, dadurch gekennzeichnet, daß die Reaktion bei 110° bis 240°C durchgeführt wird.

4. Verfahren nach Anspruch 3, dadurch gekennzeichnet, daß die Reaktion bei 200° bis 225°C durchgeführt wird.

5. Verfahren nach Anspruch 2, dadurch gekennzeichnet, daß 1,3-Diaminopropan in einem stöchiometrischen Überschuß von 3—10 molar vorliegt.

## Revendications

1. 3-Aminopropylpivalamide.

2. Procédé pour préparer le 3-aminopropylpivalamide qui comprend la mise en contact de l'acide pivalique avec le 1,3-diaminopropane, en l'absence d'un catalyseur, à des températures supérieures au point d'ébullition du 1,3-diaminopropane et l'élimination par distillation du 1,3-diaminopropane en excès.

3. Procédé selon la revendication 2, dans lequel la réaction est effectuée entre 110 et 240°C.

4. Procédé selon la revendication 3, dans lequel la réaction est effectuée entre 200 et 225°C.

5. Procédé selon la revendication 2, dans lequel le 1,3-diaminopropane est présent en excès, par rapport à la quantité stoechiométrique, selon un rapport molaire de 3 à 10.

2